# EUROPEAN PATENT APPLICATION

(11) **EP 4 822 710 A1**
(43) Date of publication of application: **14.10.2026**
(21) Application number: 25900569.2
(22) Date of filing: 02.12.2025
(51) Int. Cl.: G16H 50/30, G06N 3/0442, G06N 3/0464, G06N 3/049

(54) **HYPOGLYCEMIA EARLY-WARNING METHOD, SYSTEM AND TERMINAL, AND STORAGE MEDIUM**

(30) Priority: 03.12.2024 CN 202411762591
(71) Applicant: Changsha Sinocare, Inc., Changsha, Hunan 410205 (CN)
(72) Inventor: LI, Gaoxiang, Changsha, Hunan 410205 (CN); GAO, Fei, Changsha, Hunan 410205 (CN)
(74) Representative: Forresters IP LLP
(86) International application number: PCT/CN2025/139425
(87) International publication number: WO 2026/119143

(57) **Abstract**

The present application relates to the technical field of blood glucose prediction. Provided are a method for early warning hypoglycemia, system and terminal and a storage medium. The method includes: acquiring blood glucose time series sample data, and preprocessing same to obtain preprocessed data; constructing an LSTM-TCN model using blood glucose time series data as an input and using a predicted blood glucose value of a preset prediction horizon as an output; constructing a training data set according to the preprocessed data, and using the training data set to train the LSTM-TCN model to obtain a trained hypoglycemia prediction model; acquiring blood glucose time series monitoring data, and using the hypoglycemia prediction model to predict a blood glucose value of a preset prediction horizon, to obtain a predicted blood glucose value of the preset prediction horizon; and performing hypoglycemia early warning according to the predicted blood glucose value of the preset prediction horizon and a preset early warning mechanism. The present application can improve the predictive performance of hypoglycemia prediction models and reduce the risk caused by hypoglycemic events with limited features and without exogenous parameter input.

## Description

### Technical Field

The present application relates to the technical field of blood glucose prediction, and in particular, to a method for early warning hypoglycemia, system and terminal, and a storage medium.

### Background

Diabetes is a metabolic disease caused by insufficient insulin production or impaired insulin utilization in the human body, characterized by chronic hyperglycemia. Although diabetic patients often emphasize the challenges of daily hyperglycemia control, iatrogenic hypoglycemia frequently caused by medication control and the like during treatment also requires due attention. Studies have shown that hypoglycemia caused by oral antidiabetic drugs and insulin therapy accounts for 25% of emergency hospitalizations in the elderly, and such hypoglycemia is preventable. Acute complications of hypoglycemia may cause discomfort such as dizziness and hand tremors, and in severe cases, can even be life-threatening. Continuous Glucose Monitoring (CGM) systems are particularly important for controlling hypoglycemia risk, and the hypoglycemic event early warning enables patients to take intervention measures before hypoglycemia occurs, such as consuming fast-acting carbohydrates or stopping insulin injection, thereby avoiding the risk.

In recent years, with the advancement of ambulatory blood glucose monitoring technology, researchers have developed various hypoglycemia prediction models based on CGM data, which can predict future blood glucose data for a certain period of time using methods such as machine learning and deep learning. Existing hypoglycemia prediction models generally adopt a single-model structure and rely on a large number of features and exogenous parameters at the model input, such as age, weight, carbohydrate intake, and insulin injection dosage. Their predictive performance in terms of application and hypoglycemic events still needs to be improved.

Therefore, how to improve the predictive performance of hypoglycemia prediction models and reduce the risk caused by hypoglycemic events with limited features and without exogenous parameter input is a technical problem to be solved urgently by those skilled in the art.

### Summary

In order to solve the described technical problem, the present application provides a method for early warning hypoglycemia, which can improve the predictive performance of hypoglycemia prediction models and reduce the risk caused by hypoglycemic events with limited features and without exogenous parameter input. The present application also provides a system for early warning hypoglycemia and terminal and a storage medium, which have the same technical effects.

A first objective of the present application is to provide a method for early warning hypoglycemia.

The first objective of the present application is achieved by the following technical solution:
a method for early warning hypoglycemia, including:
acquiring blood glucose time series sample data, and preprocessing the blood glucose time series sample data to obtain preprocessed data;
constructing a Long Short-Term Memory and Temporal Convolutional Network (LSTM-TCN) model using blood glucose time series data as an input and using a predicted blood glucose value of a preset prediction horizon as an output, wherein the LSTM-TCN model includes at least two LSTM layers and one TCN layer which are sequentially connected;
constructing a training data set according to the preprocessed data, and using the training data set to train the LSTM-TCN model to obtain a trained hypoglycemia prediction model;
acquiring blood glucose time series monitoring data, and using the hypoglycemia prediction model to predict a blood glucose value of a preset prediction horizon according to the blood glucose time series monitoring data, so as to obtain a predicted blood glucose value of the preset prediction horizon; and
performing hypoglycemia early warning according to the predicted blood glucose value of the preset prediction horizon and a preset early warning mechanism.

In some embodiments of the present application, in the method for early warning hypoglycemia, preprocessing the blood glucose time series sample data to obtain the preprocessing data includes:
performing outlier removal or interpolation processing on the blood glucose time sequence sample data using a confidence interval or an n-sigma interval, so as to obtain first processed data;
performing noise reduction processing on the first processed data using an Adaptive Extended Kalman Filter (AEKF) algorithm, to obtain second processed data;
performing standardization processing on the second processed data using a z-score standardization method, to obtain third processed data; and
processing the third processed data using a sliding window method to obtain the preprocessed data.

In some embodiments of the present application, in the method for early warning hypoglycemia, the LSTM-TCN model includes a first LSTM layer, a second LSTM layer and a TCN unit that are sequentially connected, wherein the TCN unit includes at least one TCN layer that is sequentially connected, wherein
the first LSTM layer is configured to output a first temporary variable on the basis of the preprocessed data;
the second LSTM layer is configured to output an LSTM activation value on the basis of the first temporary variable; and
the TCN unit is configured to output the predicted blood glucose value of the preset prediction horizon on the basis of the LSTM activation value and a Sigmoid activation function.

In some embodiments of the present application, in the method for early warning hypoglycemia, the TCN unit is specifically configured to:
take the LSTM activation value as an input to a first TCN layer;
use at least one TCN layer to perform one-dimensional convolution on the basis of an input to the TCN layer and preset dilation factors, convolutional kernels, a sliding step and a filling step, so as to obtain an output of a one-dimensional convolutional layer;
perform feature screening and pruning on the output of the one-dimensional convolutional layer on the basis of preset hyperparameters to obtain a second temporary variable;
modify the second temporary variable using a Sigmoid activation function to obtain an output of the TCN layer; and
repeat the above steps, and in each repetition, increase the dilation factor and the filling step exponentially until a receptive field length of a one-dimensional convolutional layer of a certain TCN layer is consistent with a preset input step size, and then take a current output of the TCN layer as the predicted blood glucose value of the preset prediction horizon.

In some embodiments of the present application, in the method for hypoglycemia early warning, a maximum value of the number of TCN layers in the TCN unit is L=min(Lₘₐₓ, [log(T)/log(α)]), wherein Lₘₐₓ represents a preset maximum number of TCN layers, α represents an incremental multiple of a dilation factor, and T represents the preset input step size.

In some embodiments of the present application, in the method for early warning hypoglycemia, constructing the training data set according to the preprocessed data, and using the training data set to train the LSTM-TCN model to obtain the trained hypoglycemia prediction model, includes:
dividing the preprocessed data to obtain a training set, a validation set and a test set;
using an Adam optimizer with a set learning rate to train the LSTM-TCN model by means of the training set, adopting an early stopping mechanism, and performing parameter tuning by means of the validation set, so as to obtain a trained intermediate model;
performing performance evaluation on the intermediate model using the test set, to obtain an evaluation index; and
determining whether the evaluation index satisfies a preset requirement, and if so, using the intermediate model as a trained hypoglycemia prediction model.

In some embodiments of the present application, in the method for early warning hypoglycemia, the preset early warning mechanism includes one or more of the following early warning mechanisms:
triggering a hypoglycemia early warning when a single point within the predicted blood glucose value of the preset prediction horizon is less than a predetermined hypoglycemia threshold;
triggering a hypoglycemia early warning when predicted values at consecutive k time points within the predicted blood glucose value of the preset prediction horizon are less than the predetermined hypoglycemia threshold; and
triggering a hypoglycemia early warning when a cumulative decrease in blood glucose over consecutive k time points within the predicted blood glucose value of the preset prediction horizon exceeds a predetermined decrease threshold.

A second objective of the present application is to provide a system for early warning hypoglycemia.

The second objective of the present application is achieved by the following technical solution:
A system for early warning hypoglycemia, including:
a processing unit, configured to acquire blood glucose time series sample data, and preprocess the blood glucose time series sample data to obtain preprocessed data;
a construction unit, configured to construct an LSTM-TCN model using blood glucose time series data as an input and using a predicted blood glucose value of a preset prediction horizon as an output, wherein the LSTM-TCN model includes at least two LSTM layers and one TCN layer which are sequentially connected;
a training unit, configured to construct a training data set according to the preprocessed data, and use the training data set to train the LSTM-TCN model to obtain a trained hypoglycemia prediction model;
a prediction unit, configured to acquire blood glucose time series monitoring data, and use the hypoglycemia prediction model to predict a blood glucose value of a preset prediction horizon according to the blood glucose time series monitoring data, so as to obtain a predicted blood glucose value of the preset prediction horizon; and
an early warning unit, configured to perform hypoglycemia early warning according to the predicted blood glucose value of the preset prediction horizon and a preset early warning mechanism.

A third objective of the present application is to provide a terminal for early warning hypoglycemia.

The third objective of the present application is achieved by the following technical solution:
A terminal for early warning hypoglycemia, including: a storage medium and a processor; wherein the storage medium stores a computer execution instruction; and
the processor executes the computer execution instruction stored in the storage medium, so as to implement the foregoing method for early warning hypoglycemia.

A fourth objective of the present application is to provide a computer readable storage medium.

The fourth objective of the present application is achieved by the following technical solution:
A computer readable storage medium, wherein the computer readable storage medium stores a computer execution instruction, the computer execution instruction, when executed by a processor, cause the processor to implement the described method for early warning hypoglycemia.

The described technical solution includes: acquiring blood glucose time series sample data, and preprocessing the blood glucose time series sample data to obtain preprocessed data; constructing an LSTM-TCN model using blood glucose time series data as an input and using a predicted blood glucose value of a preset prediction horizon as an output; constructing a training data set according to the preprocessed data, and using the training data set to train the LSTM-TCN model to obtain a trained hypoglycemia prediction model; acquiring blood glucose time series monitoring data, and using the hypoglycemia prediction model to predict a blood glucose value of a preset prediction horizon according to the blood glucose time series monitoring data, so as to obtain a predicted blood glucose value of the preset prediction horizon; and performing hypoglycemia early warning according to the predicted blood glucose value of the preset prediction horizon and a preset early warning mechanism. A hypoglycemia prediction model obtained by performing training on the basis of the LSTM-TCN model predicts future blood glucose values of a preset prediction horizon only by means of blood glucose time series data, without requiring other user features or exogenous parameter inputs, and thus has more ideal guidance capability in practical applications. The LSTM-TCN model includes at least two LSTM layers and one TCN layer that are sequentially connected, such that the LSTM-TCN model integrates the long-term memory and short-term processing capabilities of LSTM with the efficient temporal feature extraction capability of TCN. The LSTM-TCN model integrates the advantages of the two, thereby not only improving the prediction performance on a hypoglycemic event, but also optimizing the time for the response of the model to the event, and increasing the duration of intervention.

In conclusion, the described technical solution can improve the predictive performance of hypoglycemia prediction models and reduce the risk caused by hypoglycemic events with limited features and without exogenous parameter input.

In addition, the LSTM-TCN model includes a first LSTM layer, a second LSTM layer and a TCN unit which are sequentially connected, wherein the TCN unit can adaptively adjust the number of layers according to data features and an input dimension; when constructing the TCN unit, the dilation factor is increased exponentially, so that the receptive field length of the one-dimensional convolutional layer matches the preset input step size; For different preset input step sizes, the number of TCN unit layers can be dynamically adjusted. Such a construction method can gradually expand the receptive field while ensuring computational efficiency, thereby enhancing the capability of a network for capturing global information.

### Brief Description of the Drawings

To describe the technical solutions in the embodiments of present application or in the existing technology more clearly, the following briefly describes the accompanying drawings required for describing the embodiments or the existing technology. Apparently, the accompanying drawings in the following description show merely some embodiments of the present application, and a person of ordinary skill in the art may still derive other accompanying drawings from these accompanying drawings without creative efforts.
Fig. 1 is a schematic flowchart of a method for early warning hypoglycemia according to some embodiments of the present application;
Fig. 2 is a schematic structural diagram of an LSTM model according to some embodiments of the present application;
Fig. 3 is a schematic structural diagram of a TCN model according to some embodiments of the present application;
Fig. 4 is a schematic structural diagram of an LSTM-TCN model according to some embodiments of the present application;
Fig. 5 is a Clarke error grid diagram of various models according to some embodiments of the present application, wherein Fig. (a) is a Clarke error grid diagram of an LSTM-TCN model, Fig. (b) is a Clarke error grid diagram of an LSTM model, Fig. 5(c) is a Clarke error grid diagram of an RNN model, and Fig. (d) is a Clarke error grid diagram of a TCN model;
Fig. 6 is a schematic structural diagram of a system for early warning hypoglycemia according to some embodiments of the present disclosure;
Fig. 7 is a schematic structural diagram of a terminal for early warning hypoglycemia according to some embodiments of the present application.

### Detailed Description of the Embodiments

In order to make the technical solutions of the present application better understood by those skilled in the art, the technical solutions in the embodiments of the present application will be clearly and completely described below. It is apparent that the described embodiments are not all embodiments but part of embodiments of the present application. All other embodiments obtained by a person skilled in the art on the basis of the embodiments in the application without creative work shall fall within the scope of protection of the present application.

In the embodiments provided in the present application, it should be understood that the disclosed method and system can be implemented in other ways. The system embodiments described above are merely exemplary. For example, division of the modules is merely logical function division, and may be other division in actual implementation. For example, division into the units is merely logical function division and may be other division in actual implementation. For example, a plurality of modules or assemblies may be combined or integrated into another system, or some features may be ignored or not executed. In addition, the coupling or direct coupling or communication connection between composition parts as shown or discussed may be indirect coupling or communication connection through some interfaces, modules or modules, and may be electrical, mechanical connection or other forms of connection.

It should be understood that if "system", "device", "unit" and/or "module" are used in the present application, it is merely a method for distinguishing different assemblies, elements, components or parts at different levels. However, if other expressions can realize the same purpose, they may be replaced by other expressions.

In addition, the terms "first" and "second" are only used for descriptive purposes, and cannot be understood as indicating or implying relative importance or implicitly indicating the number of indicated technical features. Thus, features defined by "first" and "second" may explicitly or implicitly comprise one or more of the features. In the description of the present application, "a plurality of" and "several" refer to two or more than two, unless specified otherwise.

If a flowchart is used in the present application, the flowchart is used to explain the operation performed by the system according to the embodiment of the present application. It should be understood that the preceding or subsequent operations are not necessarily performed accurately in sequence. Instead, the steps can be processed in reverse order or simultaneously. In addition, other operations can be added to these procedures, or one or more operations can be removed from these procedures.

It should be noted that in the present application, the terms "comprise", "include", or any other variations thereof are intended to cover a non-exclusive inclusion, so that an article, or a device that comprises a series of elements not only comprises those elements, but also comprises other elements that are not explicitly listed, or further comprises inherent elements of the article, or the device. Without further limitation, an element defined by a sentence "comprise a ..." does not exclude other same elements existing in an article, or a device that comprises the element.

The embodiments of the present application are written in a progressive manner.

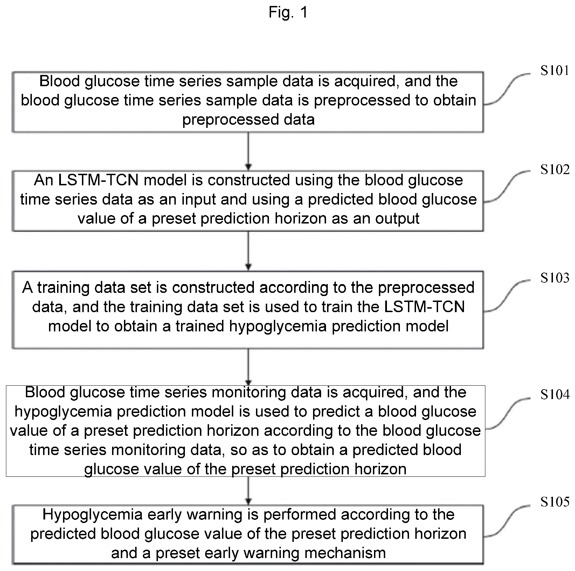

As shown in Fig. 1, an embodiment of the present application provides a hypoglycemia early warning method, comprising:
S101: blood glucose time series sample data is acquired, and the blood glucose time series sample data is preprocessed to obtain preprocessed data.

In S101, specifically, the blood glucose time series sample data may be composed of timestamped blood glucose monitoring data outputted at predetermined time intervals by existing invasive, non-invasive, electrochemical principle-based, or optical principle-based blood glucose monitoring devices, D=(t_{N}, X_{N})_{NX2}, where t_{N} represents a time stamp, and X_{N} represents continuous glucose monitoring values. For example, 120 patients diagnosed with diabetes were selected as study subjects, including 22 patients with type 1 diabetes, 97 patients with type 2 diabetes, and one patient with other specific types of diabetes. The patients use CGM devices to continuously collect blood glucose data for 15 days. The CGM sampling frequency is 3 minutes, resulting in 7,200 blood glucose values per patient, totaling 864,000 blood glucose values, which served as the blood glucose time series sample data required for model training.

In addition, in order to improve the quality of data, it is also required to preprocess the blood glucose time series sample data to obtain preprocessed data, wherein the preprocessing process may include outlier processing, noise reduction processing, standardization processing, sliding window processing, etc., and the present application is not limited thereto.

In some embodiments, the step of preprocessing the blood glucose time series sample data to obtain preprocessed data includes:
S1011: outlier removal or interpolation processing are performed on the blood glucose time sequence sample data using a confidence interval or an n-sigma interval, so as to obtain first processed data.

In S1011, specifically, a probability distribution of the blood glucose time series data is constructed, and a confidence interval (e.g., 80%, 85%, 90%, 95%, etc.) or an n-sigma interval (e.g., 2-sigma, 4-sigma, 6-sigma interval, etc.) is constructed by means of the probability distribution. Data points that do not fall within the confidence interval or the n-sigma interval are regarded as outliers, and after removing or interpolating the outliers, first processed data is obtained.

S1012: noise reduction processing is performed on the first processed data using an AEKF algorithm, to obtain second processed data.

In S1012, specifically, considering that CGM devices are susceptible to interference from confounding substances when measuring blood glucose, the collected blood glucose data contains substantial noise; an Adaptive Extended Kalman Filter (AEKF) algorithm is used to perform noise reduction on the blood glucose time series sample data collected by the CGM device; the AEKF algorithm can effectively remove high-frequency noise from the blood glucose time series sample data, and adaptively adjust the parameters of the AEKF algorithm according to data characteristics such as the noise level and outlier distribution of the first processed data, thereby obtaining more reasonable second processed data.

S1013: standardization processing is performed on the second processed data using a z-score standardization method, to obtain third processed data.

In S1013, specifically, to reduce the impact of different CGM value ranges on model training, the z-score standardization method is adopted to standardize the second processed data, so as to scale the data to the range of [-1, 1], thereby obtaining third processed data.

S1014: the third processed data is processed using a sliding window method to obtain the preprocessed data.

In S1014, specifically, the sliding window method allows for local region analysis of time series data, which facilitates capturing short-term patterns and trends in the data. By means of a sliding window method, the third processed data is processed, and short-term fluctuations and long-term information of the third processed data can be captured, so as to obtain preprocessed data, thereby facilitating a model in better understanding time sequence changes.

In the described embodiments, by using an outlier processing, an AEKF algorithm, a z-score standardization method and a sliding window method, the blood glucose time series sample data is preprocessed to obtain preprocessed data, so that the data quality can be effectively improved, and the effect of subsequent model training is facilitated to be improved.

S102: an LSTM-TCN model is constructed using the blood glucose time series data as an input and using a predicted blood glucose value of a preset prediction horizon as an output.

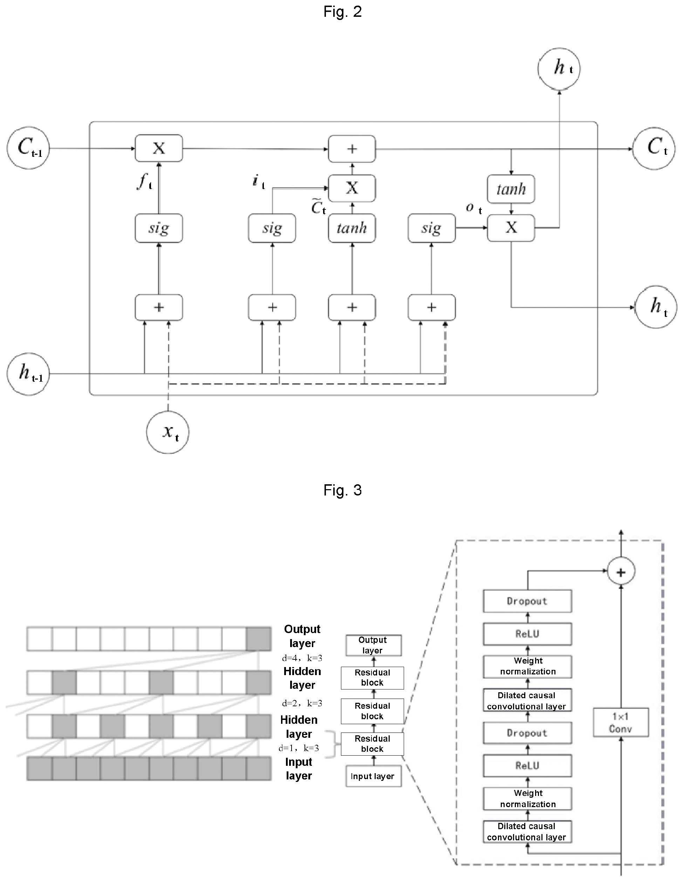

In S102, specifically, Long Short Term Memory (LSTM) was proposed by Hochreiter and Schmidhuber in 1997, and is a variant of a recurrent neural network. By introducing the memory cell C and the gating mechanism, LSTM solves the long-term memory dependency problem in recurrent neural networks and the potential issues of gradient vanishing or exploding during computation, and the structure thereof is shown in Fig. 2. The memory cell C handles long-term memory, while the hidden state h handles current and short-term information. These two variables are propagated and evolved by means of a gating mechanism. A forget gate filters old information in Cₜ₋₁ according to short-term information hₜ₋₁ passed from the previous time step t-1 and new information xₜ at the current time step, and the computational formula thereof is: ${f}_{t} = sig \left({W}_{f}⋅\left[{h}_{t - 1}, {x}_{t}\right]+{b}_{f}\right)$ (1); an input gate is responsible for calculating the new information absorbed, determining how much of the new information xₜ at the current time step needs to be passed into the long-term memory cell Cₜ, and the computational formula thereof is: ${i}_{t} = sig \left({W}_{i}⋅\left⌊{h}_{t - 1}, {x}_{t}\right⌋+{b}_{i}\right)$ (2); ${{C}^{˜}}_{t} = tanh \left({W}_{C}⋅\left[{h}_{t - 1}, {x}_{t}\right]+{b}_{c}\right)$ (3);

Then, according to the outputs of the forget gate and the input gate, the network updates the current cell state Cₜ, and the computational formula thereof is: ${C}_{t} = {f}_{t} ∗ {C}_{t - 1} + {i}_{t} ∗ {{C}^{˜}}_{t}$ (4);

The output gate filters the most effective information ht for the current time step t from the updated long-term memory t, and outputs same, wherein the computational formula thereof is: ${o}_{t} = sig \left({W}_{o}⋅\left[{h}_{t - 1}, {x}_{t}\right]+{b}_{o}\right)$ (5); ${h}_{t} = {o}_{t} ∗ tanh \left({C}_{t}\right)$ (6);

In formulas (1) to (6), sig represents the activation function Sigmoid; tanh represents the activation function tanh; *W*_{f}, *W*ᵢ, *W*_{c}, *W*ₒ represent the respective weight matrices; and b_{f}, bᵢ, b_{c}, bₒ represent the respective biases.

Specifically, Temporal Convolutional Networks (TCN) was introduced by Bai et al. in 2018 for sequence processing and is a variant of one-dimensional convolutional neural networks (CNN) specifically designed for time series prediction. The core of the TCN is to capture the time dependency in the sequence by means of a special architecture. As shown in Fig. 3, this architecture includes causal convolution, dilated convolution, and residual connections.

The causal convolution and the dilated convolution are used to extract temporal features, while the residual connections are used to reduce the risk of vanishing gradients during model training. Based on the size of the convolutional kernel, causal convolution pads 0 at the beginning of the input sequence to ensure that the model's output at time point t depends only on past and present inputs, not on future information, thereby ensuring the temporal integrity required for time series prediction. The receptive field is defined as the span of time steps of the input sequence that an output unit can perceive. Unlike CNNs that expand the receptive field by using larger convolutional kernels or increasing the number of model layers, dilated convolution increases the number of time steps it covers by introducing holes into the convolutional kernel. The size of the holes depends on the dilation rate, and an increase in the dilation rate consequently increases the receptive field. The dilated convolution enables the model to acquire a wider range of historical information and learn long-term dependencies without increasing the computational parameters or cost of the model. The residual connection adds an input of one network layer to an output of another network layer, alleviating the vanishing gradient problem and enhancing the learning capability of the model. This design not only improves the training efficiency of deep networks, but also ensures the stability of the model when processing long sequences.

In this step, in order to combine the advantages of LSTM in capturing long-term dependencies and TCN in rapidly extracting local features, the LSTM network and the TCN network are combined in advance, so as to construct an LSTM-TCN model using the blood glucose time series data as an input and using a predicted blood glucose value of a preset prediction horizon as an output, wherein the LSTM-TCN model includes at least two LSTM layers and one TCN layer which are sequentially connected. The predicted blood glucose value of the preset prediction horizon refers to a sequence of blood glucose predictions over a future period of time. The preset prediction horizon can be 15 minutes, 30 minutes, 60 minutes, or other preset step size. On the basis of the above model architecture design, the LSTM-TCN model integrates the long-term memory capability of LSTM and the efficient temporal feature extraction capability of TCN. The LSTM-TCN model combines the advantages of both. The hypoglycemia prediction model trained on the basis of the LSTM-TCN model not only improves the prediction performance on hypoglycemic events but also optimizes the model's response time to events, increasing the intervention duration, thereby providing more ideal guidance capability in practical applications. Furthermore, the LSTM-TCN model only requires the input of time-stamped blood glucose data, without the need for other features or exogenous parameters, thus providing more ideal guidance capability in practical applications.

In some embodiments of the present application, the LSTM-TCN model includes a first LSTM layer, a second LSTM layer, and a TCN unit that are sequentially connected, wherein the first LSTM layer is configured to output a first temporary variable on the basis of the preprocessed data;
the second LSTM layer is configured to output an LSTM activation value on the basis of the first temporary variable; and
the TCN unit is configured to output the predicted blood glucose value of the preset prediction horizon on the basis of the LSTM activation value and a Sigmoid activation function.

Specifically, the first LSTM layer takes the preprocessed data as an input, performs calculations according to the computational process in step S102, and then obtains the most effective information hₜ at the current time step t, which is output as the first temporary variable. The second LSTM layer takes the first temporary variable as an input, performs calculations according to the computational process in step S102, and obtains the most effective information hₜ at the current time step t, which is outputted as an LSTM activation value.

The TCN unit includes at least one TCN layer connected in sequence. Each TCN layer extracts time series features by means of multiple convolutional kernels and pooling operations, and is mainly composed of a dilated causal convolutional layer, a residual module, a pooling layer, and a fully connected layer. The TCN unit extracts features from the LSTM activation values of the data sequence, and then applies the Sigmoid activation function to obtain the predicted blood glucose values of the preset prediction step size output by the TCN unit.

The TCN unit is specifically configured to:
take the LSTM activation value as an input to a first TCN layer;
use at least one TCN layer to perform one-dimensional convolution on the basis of an input to the TCN layer and preset dilation factors, convolution kernels, a sliding step and a filling step, so as to obtain an output of a one-dimensional convolutional layer;
perform feature screening and pruning on the output of the one-dimensional convolutional layer on the basis of preset hyperparameters to obtain a second temporary variable;
modify the second temporary variable using a Sigmoid activation function to obtain an output of the TCN layer; and
repeat the above steps, and in each repetition, increase the dilation factor and the filling step exponentially until a receptive field length of a one-dimensional convolutional layer of a certain TCN layer is consistent with a preset input step size, and then take a current output of the TCN layer as the predicted blood glucose value of the preset prediction horizon.

It should be noted that when the TCN unit comprises only one TCN layer, the TCN layer may be reused to re-execute the aforementioned process, that is, repeating the process of performing processing by using at least one TCN layer and on the basis of the input to the TCN layer, the preset dilation factor, the convolution kernel, the sliding step, and the filling step, to ultimately obtain the output of the TCN layer. The output of the TCN layer from the previous round of computation serves as the input to the TCN layer in the subsequent round of computation, until the receptive field length of the one-dimensional convolutional layer of the TCN layer matches the preset input step size, at that point, the current output of the TCN layer is used as the predicted blood glucose value of the preset prediction step size. When the TCN unit comprises a plurality of TCN layers, the output of the previous TCN layer serves as the input to the subsequent TCN layer. The plurality of TCN layers sequentially and cyclically execute the aforementioned process, i.e. repeating the process of performing processing by using at least one TCN layer and on the basis of the input to the TCN layer, the preset dilation factor, the convolutional kernel, the sliding step and the filling step, to ultimately obtain the output of the TCN layer, until the receptive field length of the one-dimensional convolutional layer of one of the TCN layers among the plurality of TCN layers matches the preset input step size; at that point, the current output of the TCN layer is used as the predicted blood glucose value of the preset prediction step size.

In some embodiments of the present application, the TCN unit can adaptively adjust the number of layers according to the data features and input dimensions; when constructing the TCN unit, the dilation factor is increased exponentially, so that the receptive field length of the one-dimensional convolutional layer matches the preset input step size. For different preset input step sizes, the number of TCN unit layers can be dynamically adjusted. Such a construction method can gradually expand the receptive field while ensuring computational efficiency, thereby enhancing the capability of a network for capturing global information. Specifically, the number of TCN layers is adjusted adaptively as follows:
For the TCN layer of the l^{th} layer, the receptive field length thereof is Rₗ=Rₗ₋₁+(kₗ-1) dₗ₋₁ where Rₗ is the receptive field length of the TCN layer of the l^{th} layer , Rₗ₋₁ is the receptive field length of the TCN layer of the (l-1)^{th} layer, kₗ is the convolutional kernel size of the TCN layer of the l^{th} layer, and dₗ₋₁ is the dilation factor of the TCN layer of the (l-1)^{th} layer. On the basis of the aforementioned receptive field length calculation formula and the preset input step size T, the maximum number of TCN layers in a dynamically adjustable TCN unit can be constructed as: L=min(Lₘₐₓ, [log(T)/log(α)]), where Lₘₐₓ represents the preset maximum number of TCN layers, and α represents incremental multiples of dilation factors; in some embodiments, the dilation factor is incremented exponentially, generally, the incremental multiplier α of the dilation factor is set to 2.

In other embodiments of the present application, the LSTM-TCN model includes an input layer, a first LSTM layer, a second LSTM layer, a first TCN layer, a second TCN layer, a third TCN layer, a Dropout layer, an MLP layer, and an output layer which are connected in sequence.

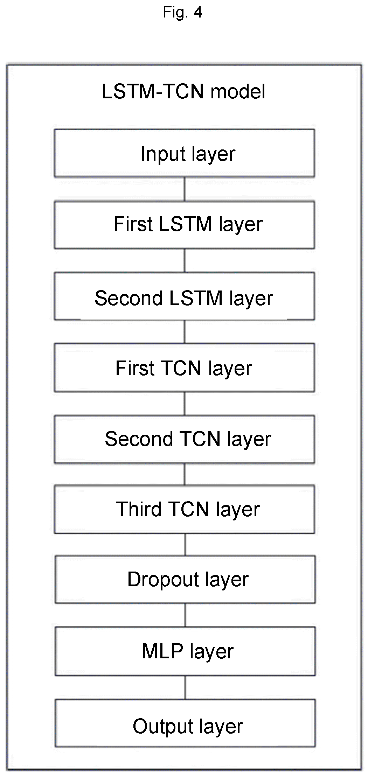

Specifically, as illustrated in Fig. 4, the input layer is used for receiving blood glucose time series data as the model input. The model input is specifically an N x KT dimensional matrix, where N is the number of samples, K is the number of features, and T is the input step size. Here, the input step size can be understood as the length of the sliding window, i.e., continuous blood glucose data of a length T are used for blood glucose prediction of a predefined prediction horizon. The first LSTM layer and the second LSTM layer are used for processing sequence data and extracting key long-term information; preferably, the first LSTM layer and the second LSTM layer both use an LSTM layer containing 64 neurons; these information is then passed into three TCN layers, i.e., the first TCN layer, the second TCN layer, and the third TCN layer, to further extract the temporal features that are most important for short-term correlation. Preferably, the first TCN layer, the second TCN layer, and the third TCN layer each have a convolutional kernel size of 3, with the number of filters being 10, 32, and 64, respectively, and the dilation rates being 1, 2, and 4, respectively. Causal convolution is used to ensure that the output depends only on current and past inputs. The Dropout layer is a structure that applies dropout technology to neural networks. By randomly discarding some neurons during the training process, it achieves regularization. In addition, the Dropout layer has the advantage of reducing model complexity, which facilitates accelerating the model training process. Preferably, to prevent overfitting in model training, the dropout rate of the Dropout layer is set to 10%. The Multi-Layer Perceptron (MLP) layer is a specific type of artificial neural network composed of multiple neurons, and used to connect the convolutional layers and the output layer. The output layer uses the sigmoid activation function to output the predicted blood glucose value of a preset prediction horizon.

S103: a training data set is constructed according to the preprocessed data, and the training data set is used to train the LSTM-TCN model to obtain a trained hypoglycemia prediction model.

In S103, specifically, the preprocessed data are randomly sorted as a per-subject basis, and then divided according to a set ratio to construct a training dataset consisting of a training set, a validation set and a test set for model training. For example, the first 80% of the data is assigned as the training set, the middle 10% as the validation set, and the last 10% as the test set, which are respectively used for subsequent model training, tuning, and evaluation. During the training process of the LSTM-TCN model, the Mean Absolute Error (MAE) can be selected as the loss function, and Adam as the optimizer. The training is performed using a mini-batch approach, with a batch size set to 64 and the number of training set to 100. To prevent overfitting, an early stopping mechanism is employed to obtain a trained hypoglycemia prediction model, and the present application is not limited thereto.

S104: blood glucose time series monitoring data is acquired, and the hypoglycemia prediction model is used to predict a blood glucose value of a preset prediction horizon according to the blood glucose time series monitoring data, so as to obtain a predicted blood glucose value of the preset prediction horizon.

In S104, specifically, blood glucose time series monitoring data of a preset step size can be obtained from the person under glucose monitoring using a CGM device; and the blood glucose time series monitoring data is then input into the hypoglycemia prediction model to obtain a predicted blood glucose value of the preset prediction horizon. Taking intervention measures 20-30 minutes before the occurrence of a hypoglycemic event can effectively reduce the risk of complications. Furthermore, 30 minutes represents the optimal trade-off point between prediction error and prediction effectiveness. Therefore, the prediction horizon (PH) of the hypoglycemia prediction model can be preferably set to 30 minutes. In other embodiments, the blood glucose time series monitoring data may also be preprocessed before being input into the hypoglycemia prediction model for prediction. The preprocessing procedure may refer to step S101 described above, which is not specifically limited in the present application.

S105: hypoglycemia early warning is performed according to the predicted blood glucose value of the preset prediction horizon and a preset early warning mechanism.

In S105, specifically, the preset early warning mechanism may include one or more of the following warning mechanisms: (1) triggering a hypoglycemia early warning when a single point within the predicted blood glucose value of the preset prediction horizon is less than a predetermined hypoglycemia threshold; (2) triggering a hypoglycemia early warning when predicted values at consecutive k time points within the predicted blood glucose value of the preset prediction horizon are less than a predetermined hypoglycemia threshold; and (3) triggering a hypoglycemia early warning when a cumulative decrease in blood glucose over consecutive k time points within the predicted blood glucose value of the preset prediction horizon exceeds a predetermined decrease threshold. The predetermined hypoglycemia threshold, the time instant k, and the predetermined decrease threshold can be set based on actual application requirements. For example, when the predicted blood glucose value is lower than 70 mg/dL for 15 consecutive minutes, a hypoglycemia early warning is triggered so as to take an intervention measure to effectively avoid the risk of complications.

In recent years, with the advancement of ambulatory blood glucose monitoring technology, researchers have developed various hypoglycemia prediction models based on CGM data, which can predict future blood glucose data for a certain period of time using methods such as machine learning and deep learning. Existing hypoglycemia prediction models generally adopt a single-model structure and rely on a large number of features and exogenous parameters at the model input, such as age, weight, carbohydrate intake, and insulin injection dosage. Their predictive performance in terms of application and hypoglycemic events still needs to be improved.

In the described embodiments, by acquiring blood glucose time series sample data, and preprocessing the blood glucose time series sample data to obtain preprocessed data; constructing an LSTM-TCN model using the blood glucose time series data as an input and using a predicted blood glucose value of a preset prediction horizon as an output; constructing a training data set according to the preprocessed data, and using the training data set to train the LSTM-TCN model to obtain a trained hypoglycemia prediction model; acquiring blood glucose time series monitoring data, and using the hypoglycemia prediction model to predict a blood glucose value of a preset prediction horizon according to the blood glucose time series monitoring data, so as to obtain a predicted blood glucose value of the preset prediction horizon; and performing hypoglycemia early warning according to the predicted blood glucose value of the preset prediction horizon and a preset early warning mechanism, the LSTM-TCN model includes at least two LSTM layers and one TCN layer that are sequentially connected, such that the LSTM-TCN model integrates the long-term memory and short-term processing capabilities of LSTM with the efficient temporal feature extraction capability of TCN. The LSTM-TCN model integrates the advantages of the two. The hypoglycemia prediction model trained on the basis of the LSTM-TCN model not only improves the prediction performance on a hypoglycemic event, but also optimizes the time for the response of the model to the event, and increases the duration of intervention. Furthermore, the described embodiment only requires the input of time-stamped blood glucose data, without the need for other features or exogenous parameters, thus providing more ideal guidance capability in practical applications. In conclusion, the embodiments can improve the predictive performance of hypoglycemia prediction models and reduce the risk caused by hypoglycemic events with limited features and without exogenous parameter input.

In other embodiments of the present application, one implementation of the steps of constructing a training data set according to the preprocessed data, and using the training data set to train the LSTM-TCN model to obtain a trained hypoglycemia prediction model specifically includes:
S201: the preprocessed data is divided to obtain a training set, a validation set and a test set.

In S201, specifically, the preprocessed data are randomly sorted as a per-subject basis, and then divided according to a set ratio to construct a training dataset consisting of a training set, a validation set and a test set for model training. For example, the first 80% of the data is assigned as the training set, the middle 10% as the validation set, and the last 10% as the test set, which are respectively used for subsequent model training, tuning, and evaluation.

S202: an Adam optimizer with a set learning rate is used to train the LSTM-TCN model by means of the training set, an early stopping mechanism is adopted, and parameter tuning is performed by means of the validation set, so as to obtain a trained intermediate model.

In S202, specifically, the MAE is selected as the loss function, and an Adam optimizer with a dynamically adjusted learning rate is used. The training is performed using a mini-batch approach, with a batch size set to 64 and the number of training set to 100. An early stopping mechanism is adopted. Based on the above mechanisms, the LSTM-TCN model is trained using the training set.

The validation set is used to evaluate the model performance of the trained LSTM-TCN model and to adjust the hyperparameters, such as learning rate and batch size, of the model. If the total validation loss does not change or increases for several consecutive epochs, training is stopped, thereby obtaining a trained intermediate model.

The early stopping mechanism can terminate training after 15 epochs loss function without decline under the condition that there is no substantial reduction in the validation loss, and the early stopping mechanism can accelerate the training process and preserve the optimal weights obtained during training. The update rule of the Adam optimizer is as follows:
Compute the gradient gₜ of the loss function L(θ) with respect to the model parameter θ: ${g}_{t} = L \left(θ\right) / θ$ (7); where t represents the current time step.

Based on the gradient gₜ of the current model parameter θ, the first-order moment estimate mₜ and the second-order moment estimate vₜ of the gradient gₜ are calculated, and the calculation formulas are: ${m}_{t} = {β}_{1} {m}_{t - 1} + \left(1-{β}_{1}\right) {g}_{t}$ (8); ${v}_{t} = {β}_{2} {v}_{t - 1} + \left(1-{β}_{2}\right) {g}_{t}^{2}$ (9); wherein, β₁ represents the first-order momentum decay rate, and β₂ represents the second-order momentum decay rate; t-1 represents the previous time step before the current time step t.

Perform bias correction on the first-order moment estimate mt and the second-order moment estimate vt to obtain the corrected first-order moment estimate *m̂ₜ* and the corrected second-order moment estimate *v̂ₜ*. The calculation formulas are: ${\hat{m}}_{t} = \frac{{m}_{t}}{1 - {β}_{1}^{t}}$ (10); ${\hat{v}}_{t} = \frac{{v}_{t}}{1 - {β}_{2}^{t}}$ (11);

Based on the corrected first-order moment estimate *m̂ₜ* and the corrected second-order moment estimate *v̂ₜ,* the model parameter is updated, specifically as follows: ${θ}_{t + 1} = {θ}_{t} - α \frac{{\hat{m}}_{t}}{\sqrt{{\hat{v}}_{t}} + ε}$ (12);

Wherein θₜ₊₁ and θₜ represent the model parameter at the next time step of the current time step t and the model parameter at the current time step t, respectively; αrepresents the learning rate, and *ε* represents a parameter to prevent division by zero.

By means of bias correction, the Adam optimizer can resize its parameter updates more quickly, thereby accelerating the initial learning process and improving the overall optimization efficiency.

S203: performance evaluation is performed on the intermediate model using the test set, to obtain an evaluation index.

In S203, specifically, the test set is used to ultimately test the model performance. Based on the model prediction results on the test set, the evaluation metrics of the intermediate model can be calculated. These evaluation metrics include one or more of the following: Recall, Precision, F1-score, Time Gain (TG), average daily false alarm rate, and Clarke Error Grid Analysis (CEG).

In some embodiments of the present application, a hypoglycemic event is considered to occur when CGM reading falls below 70 mg/dL for 15 consecutive minutes, and the hypoglycemic event is considered to end when CGM reading rises above 70 mg/dL for 15 consecutive minutes. Therefore, the 15th minute of the hypoglycemic event is taken as a discrimination point (h). Each time the model predicts a hypoglycemic event (p), the early warning is triggered only once. In order to effectively evaluate an early warning algorithm, all early warning points are taken as (p), and a corresponding early warning classification method is as follows:
True Positive (TP): if an early warning point p is triggered between half an hour and three minutes before the discrimination point h, it is considered a successful early warning;
Late Alarm (LA): if an early warning point p coincides with the occurrence period of a hypoglycemic event, it is considered a late alarm;
False Positive (FP): if no hypoglycemic event occurs within half an hour after an early warning point p is triggered, it is considered as false positive, i.e., an erroneous early warning;
False Negative (FN): if a hypoglycemic event is not classified as True Positive (TP) and Late Alarm (LA), it is considered false negative, i.e., a missed early warning.

To avoid duplicate classification, each early warning point is classified only once and according to the classification order mentioned above. According to the warning mechanism, if the CGM reading is already lower than the threshold, no early warning is triggered. Therefore, in practical applications, an alarm classified as LA will not be issued. It should not be regarded as FP, but rather counted as FN. Since hypoglycemic events are low-probability events and constitute imbalanced data, recall, precision, and F1 score are preferably selected as the evaluation metrics for the hypoglycemia prediction model.

On the basis of the model prediction results on the test set, the recall, precision and F1 score can be calculated according to Equations (13) to (15), specifically as follows: $Recall = \frac{TP}{TP + FN}$ (13); $Precision = \frac{TP}{TP + FP}$ (14); $F 1 - score = 2 ∗ \frac{Recall ∗ Precision}{Recall + Precision}$ (15);

Recall measures the probability of successful early warning among all hypoglycemic events. Precision measures the probability of correct warning among all issued warnings. The F1 score is the harmonic mean of the first two metrics, providing a more comprehensive perspective when both recall and precision are important.

In some embodiments of the present application, to evaluate the practicality of the early warning, time gain and average daily false alarm rate are also introduced as evaluation metrics. Time gain measures the time interval between an early warning and a hypoglycemic event, reflecting the response time provided to the patient by the early warning. To evaluate the credibility of the early warning and the use experience of the patient, the average daily false alarm rate is introduced, which measures the average probability of false alarms received by the patient per day during CGM use.

In other embodiments of the present application, a Clarke Error Grid can also be used to evaluate the blood glucose prediction effect of the model. Specifically, the predicted blood glucose values are paired with the actual blood glucose values and divided into five zones according to the error, labeled A to E, where the harmfulness increases progressively. Zone A represents clinically accurate estimates that can correctly guide clinical treatment, while Zone B represents clinically acceptable estimates with a deviation exceeding 20%. Zone C represents results that may lead to potentially dangerous overtreatment, such as excessive insulin injection. Zone D represents severe hypoglycemic or hyperglycemic episodes that remain undetected, while Zone E represents completely erroneous and opposite determinations, such as identifying hypoglycemia as hyperglycemia. The more values that fall into Zone A and Zone B, and the higher their proportion, the greater the clinical utility of the prediction.

S204: it is determined whether the evaluation index satisfies a preset requirement, and if so, the intermediate model is used as a trained hypoglycemia prediction model.

In S204, specifically, the values of the evaluation metrics may be compared with a preset threshold range. If the values of all evaluation metrics fall within a preset threshold range, the evaluation metrics can be considered to meet the preset requirements, and the intermediate model is then taken as the trained hypoglycemia prediction model.

In some embodiments of the present application, the LSTM-TCN model is trained, validated, and tested using the training set, validation set, and test set, which effectively ensures the training effectiveness of the hypoglycemia prediction model and improves the prediction performance of the hypoglycemia prediction model.

In practical applications, in order to compare the prediction performance of an LSTM-TCN model with that of a monomer model, an LSTM model, a TCN model, a Recurrent Neural Network (RNN) model and an LSTM-TCN model are used to retrospectively predict hypoglycemic events for 12 diabetic patients; and preferably, the LSTM-TCN model comprises an input layer, two LSTM layers, three TCN layers, a Drop layer, an MLP layer and an output layer which are sequentially connected. According to the results in Table 1, the LSTM-TCN model outperforms the standalone LSTM model or TCN model in terms of evaluation metrics. The LSTM-TCN model achieves a recall of 76.20%, outperforming the LSTM model's 74.00% and the TCN model's 72.04%, and achieves a precision of 78.43%, which is also superior to the other models. This indicates that compared to other monomer models, the LSTM-TCN model is more capable of correctly early warning of hypoglycemic events, reducing missed early warnings and lowering the risk of undetected hypoglycemia. Meanwhile, the LSTM-TCN model achieves an F1 score of 74.64%, which is higher than those of the LSTM model and the TCN model, reflecting the model's balance in performance.

**Table 1 Statistical table of hypoglycemia event prediction performance**

| Model | Hypoglycemic Event Prediction Performance | | | | |
|---|---|---|---|---|---|
| | Precision | Recall | F1 Score | Time Gain | Average Daily False Alarm Rate |
| LSTM-TCN | 78.43% | 76.20% | 74.64% | 11.56±1.95 | 13.33% |
| LSTM | 64.71% | 74.00% | 69.55% | 6.28±0.65 | 42.22% |
| TCN | 33.33% | 72.04% | 58.91% | 4.48±0.90 | 10.00% |
| RNN | 25.49% | 58.75% | 49.84% | 4.8±1.02 | 13.33% |

In terms of time gain, the LSTM-TCN model achieves a leading result of 11.56 minutes on average, which is the highest time gain among all models, providing patients with a longer intervention time. Meanwhile, the LSTM-TCN model retains the advantage of the TCN model's low daily false alarm rate, with patients potentially receiving only two unnecessary early warnings within 15 days.

**Table 2 Proportion of each CEG zone for each model under a 30-minute prediction horizon**

| Model | CE | | | zone | | |
|---|---|---|---|---|---|---|
| | A | B | C | D | E | A+B |
| LSTM-TCN | 92.12% | 7.44% | 0 | 0.51% | 0 | 99.56% |
| LSTM | 91.20% | 8.25% | 0 | 0.55% | 0 | 99.45% |
| TCN | 91.33% | 8.06% | 0 | 0.60% | 0 | 99.39% |
| RNN | 91.14% | 8.34% | 0 | 0.51% | 0 | 99.49% |

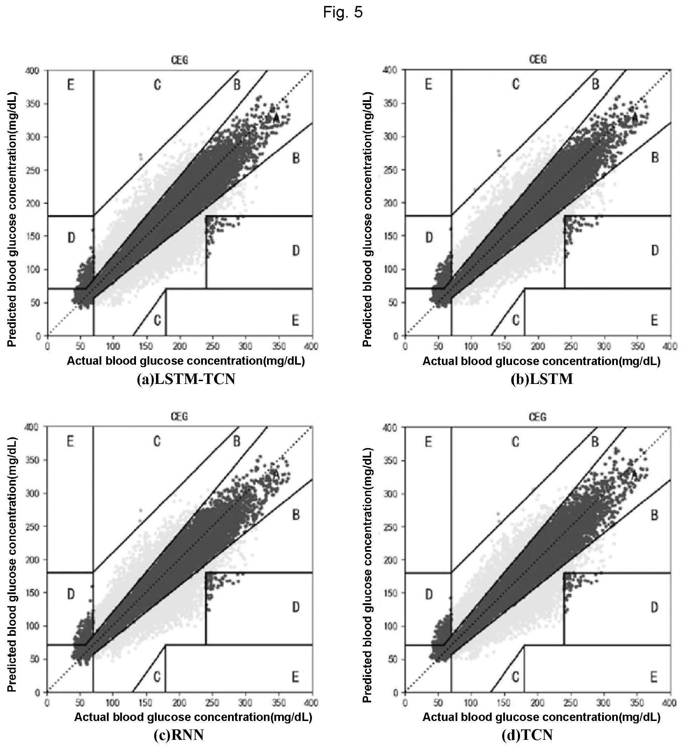

Fig. 5 and Table 2 show the CEG results of predicting the blood glucose of 14 patients over the next 30 minutes using the above method. The proportion of Zones A of the LSTM-TCN model reaches 92.12%, which is higher than those of the LSTM model (91.2%), the TCN model (91.33%), and the RNN model (91.14%). Meanwhile, the proportion of Zones A+B is also the highest among all models, reaching 99.56%, indicating that the LSTM-TCN model outperforms other models in terms of clinical accuracy.

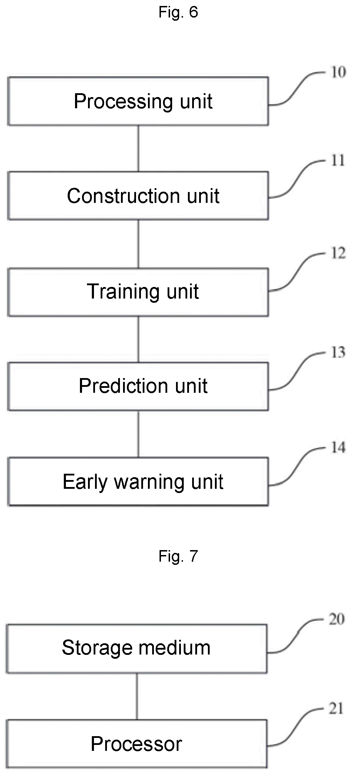

As shown in Fig. 6, in another embodiment of the present application, a system for early warning hypoglycemia is further provided, including:
a processing unit 10, configured to acquire blood glucose time series sample data, and preprocess the blood glucose time series sample data to obtain preprocessed data;
a construction unit 11, configured to construct an LSTM-TCN model using blood glucose time series data as an input and using a predicted blood glucose value of a preset prediction horizon as an output, wherein the LSTM-TCN model includes at least two LSTM layers and one TCN layer which are sequentially connected;
a training unit 12, configured to construct a training data set according to the preprocessed data, and use the training data set to train the LSTM-TCN model to obtain a trained hypoglycemia prediction model;
a prediction unit 13, configured to acquire blood glucose time series monitoring data, and use the hypoglycemia prediction model to predict a blood glucose value of a preset prediction horizon according to the blood glucose time series monitoring data, so as to obtain a predicted blood glucose value of the preset prediction horizon; and
an early warning unit 14, configured to perform hypoglycemia early warning according to the predicted blood glucose value of the preset prediction horizon and a preset early warning mechanism.

In other embodiments of the present application, when preprocessing the blood glucose time series sample data to obtain preprocessed data, the processing unit 10 is specifically used for:
performing outlier removal or interpolation processing on the blood glucose time sequence sample data using a confidence interval or an n-sigma interval, so as to obtain first processed data;
performing noise reduction processing on the first processed data using an AEKF algorithm, to obtain second processed data;
performing standardization processing on the second processed data using a z-score standardization method, to obtain third processed data; and
processing the third processed data using a sliding window method to obtain the preprocessed data.

In other embodiments of the present application, the LSTM-TCN model includes a first LSTM layer, a second LSTM layer and a TCN unit that are sequentially connected, wherein the TCN unit includes at least one TCN layer that is sequentially connected, wherein
the first LSTM layer is configured to output a first temporary variable on the basis of the preprocessed data;
the second LSTM layer is configured to output an LSTM activation value on the basis of the first temporary variable; and
the TCN unit is configured to output the predicted blood glucose value of the preset prediction horizon on the basis of the LSTM activation value and a Sigmoid activation function.

In other embodiments of the present application, the TCN unit is specifically configured to:
take the LSTM activation value as an input to a first TCN layer;
use at least one TCN layer to perform one-dimensional convolution on the basis of an input to the TCN layer and preset dilation factors, convolutional kernels, a sliding step and a filling step, so as to obtain an output of a one-dimensional convolutional layer;
perform feature screening and pruning on the output of the one-dimensional convolutional layer on the basis of preset hyperparameters to obtain a second temporary variable;
modify the second temporary variable using a Sigmoid activation function to obtain an output of the TCN layer; and
repeat the above steps, and in each repetition, increase the dilation factor and the filling step exponentially until a receptive field length of a one-dimensional convolutional layer of a certain TCN layer is consistent with a preset input step size, and then take a current output of the TCN layer as the predicted blood glucose value of the preset prediction horizon.

In other embodiments of the application, a maximum value of the number of TCN layers in the TCN unit is L=min(Lₘₐₓ, [log(T)/log(α)]), wherein Lₘₐₓ represents a preset maximum number of TCN layers, α represents an incremental multiple of a dilation factor, and T represents the preset input step size.

In other embodiments of the present application, when constructing the training data set according to the preprocessed data, and using the training data set to train the LSTM-TCN model to obtain the trained hypoglycemia prediction model, the training unit 12 is specifically used for:
dividing the preprocessed data to obtain a training set, a validation set and a test set;
using an Adam optimizer with a set learning rate to train the LSTM-TCN model by means of the training set, adopting an early stopping mechanism, and performing parameter tuning by means of the validation set, so as to obtain a trained intermediate model;
performing performance evaluation on the intermediate model using the test set, to obtain an evaluation index; and
determining whether the evaluation index satisfies a preset requirement, and if so, using the intermediate model as a trained hypoglycemia prediction model.

In other embodiments of the present disclosure, the preset early warning mechanism includes one or more of the following warning mechanisms:
triggering a hypoglycemia early warning when a single point within the predicted blood glucose value of the preset prediction horizon is less than a predetermined hypoglycemia threshold;
triggering a hypoglycemia early warning when predicted values at consecutive k time points within the predicted blood glucose value of the preset prediction horizon are less than the predetermined hypoglycemia threshold; and
triggering a hypoglycemia early warning when a cumulative decrease in blood glucose over consecutive k time points within the predicted blood glucose value of the preset prediction horizon exceeds a predetermined decrease threshold.

As shown in Fig. 7, in another embodiment of the present application, a terminal for early warning hypoglycemia is further provided, including: a storage medium 20 and a processor 21;
wherein the storage medium 20 stores a computer execution instruction; and the processor 21 executes the computer execution instruction stored in the storage medium 20, so as to implement the foregoing method for early warning hypoglycemia.

The processor 21 may include one or more processing cores. The processor 21 calls data stored in the storage medium 20 by running or executing an instruction, a program, a code set or an instruction set stored in the storage medium 20, so as to execute various functions and process data of the present application. The processor 21 may be at least one of an application specific integrated circuit, a digital signal processor, a digital signal processing apparatus, a programmable logic apparatus, a field programmable gate array, a central processing unit, a controller, a microcontroller, and a microprocessor. It can be understood that, for different devices, the electronic device for implementing the functions of the processor 21 may be other electronic devices.

The storage medium 20 may be configured to store an instruction, a program, code, a code set, or an instruction set. The storage medium 20 may comprise a program storage area and a data storage area, wherein the program storage area may store an instruction for realizing an operating system, an instruction for at least one function, an instruction for realizing the described method for early warning hypoglycemia, etc.; the data storage area may store data involved in the described method for early warning hypoglycemia, etc.

Another embodiment of the present application further provides a computer readable storage medium, wherein the computer readable storage medium stores a computer execution instruction, the computer execution instruction, when executed by a processor, cause the processor to implement the described method for early warning hypoglycemia.

The computer readable storage medium may be any medium that can store program code, such as a USB flash disk, a removable hard disk, a read-only memory, a random access memory, or an optical disk.

The foregoing descriptions of the disclosed embodiments enable persons skilled in the art to implement or use the present disclosure. Various modifications to these embodiments will be readily apparent to those skilled in the art. The general principles defined herein may be implemented in other embodiments, without departing from the spirit or scope of the present application. Therefore, the present application will not be limited to the embodiments described herein, but should be in accordance with the broadest scope consistent with the principle and novel features disclosed herein.

## Claims

1. A method for early warning hypoglycemia, comprising:
acquiring blood glucose time series sample data, and preprocessing the blood glucose time series sample data to obtain preprocessed data;
constructing a Long Short-Term Memory and Temporal Convolutional Network, LSTM-TCN, model using blood glucose time series data as an input and using a predicted blood glucose value of a preset prediction horizon as an output, wherein the LSTM-TCN model comprises at least two LSTM layers and one TCN layer which are sequentially connected;
constructing a training data set according to the preprocessed data, and using the training data set to train the LSTM-TCN model to obtain a trained hypoglycemia prediction model;
acquiring blood glucose time series monitoring data, and using the hypoglycemia prediction model to predict a blood glucose value of a preset prediction horizon according to the blood glucose time series monitoring data, to obtain a predicted blood glucose value of the preset prediction horizon; and
performing hypoglycemia early warning according to the predicted blood glucose value of the preset prediction horizon and a preset early warning mechanism.

2. The method as claimed in claim 1, wherein the preprocessing the blood glucose time series sample data to obtain the preprocessing data comprises:
performing outlier removal or interpolation processing on the blood glucose time sequence sample data using a confidence interval or an n-sigma interval, to obtain first processed data;
performing noise reduction processing on the first processed data using an Adaptive Extended Kalman Filter, AEKF, algorithm, to obtain second processed data;
performing standardization processing on the second processed data using a z-score standardization method, to obtain third processed data; and
processing the third processed data using a sliding window method to obtain the preprocessed data.

3. The method as claimed in claim 1, wherein the LSTM-TCN model comprises a first LSTM layer, a second LSTM layer and a TCN unit that are sequentially connected, wherein the TCN unit comprises at least one TCN layer that is sequentially connected, wherein
the first LSTM layer is configured to output a first temporary variable on the basis of the preprocessed data;
the second LSTM layer is configured to output an LSTM activation value on the basis of the first temporary variable; and
the TCN unit is configured to output the predicted blood glucose value of the preset prediction horizon on the basis of the LSTM activation value and a Sigmoid activation function.

4. The method as claimed in claim 3, wherein the TCN unit is specifically configured to:
take the LSTM activation value as an input to a first TCN layer;
use at least one TCN layer to perform one-dimensional convolution on the basis of an input to the TCN layer and preset dilation factors, convolutional kernels, a sliding step and a filling step, to obtain an output of a one-dimensional convolutional layer;
perform feature screening and pruning on the output of the one-dimensional convolutional layer on the basis of preset hyperparameters to obtain a second temporary variable;
modify the second temporary variable using a Sigmoid activation function to obtain an output of the TCN layer; and
repeat the above steps, and in each repetition, increase the dilation factor and the filling step exponentially until a receptive field length of a one-dimensional convolutional layer of a certain TCN layer is consistent with a preset input step size, and then take a current output of the TCN layer as the predicted blood glucose value of the preset prediction horizon.

5. The method as claimed in claim 4, wherein a maximum value of the number of TCN layers in the TCN unit is L=min(Lₘₐₓ, [log(T)/log(α)]), wherein Lₘₐₓ represents a preset maximum number of TCN layers, α represents an incremental multiple of a dilation factor, and T represents the preset input step size.

6. The method as claimed in claim 1, wherein constructing the training data set according to the preprocessed data, and using the training data set to train the LSTM-TCN model to obtain the trained hypoglycemia prediction model, comprise:
dividing the preprocessed data to obtain a training set, a validation set and a test set;
using an Adam optimizer with a set learning rate to train the LSTM-TCN model by means of the training set, adopting an early stopping mechanism, and performing parameter tuning by means of the validation set, to obtain a trained intermediate model;
performing performance evaluation on the intermediate model using the test set, to obtain an evaluation index; and
determining whether the evaluation index satisfies a preset requirement, and if so, using the intermediate model as a trained hypoglycemia prediction model.

7. The method as claimed in claim 1, wherein the preset early warning mechanism comprises one or more of the following early warning mechanisms:
triggering a hypoglycemia early warning when a single point within the predicted blood glucose value of the preset prediction horizon is less than a predetermined hypoglycemia threshold;
triggering a hypoglycemia early warning when predicted values at consecutive k time points within the predicted blood glucose value of the preset prediction horizon are less than the predetermined hypoglycemia threshold; and
triggering a hypoglycemia early warning when a cumulative decrease in blood glucose over consecutive k time points within the predicted blood glucose value of the preset prediction horizon exceeds a predetermined decrease threshold.

8. A system for early warning hypoglycemia, comprising:
a processing unit, configured to acquire blood glucose time series sample data, and preprocess the blood glucose time series sample data to obtain preprocessed data;
a construction unit, configured to construct a Long Short-Term Memory and Temporal Convolutional Network, LSTM-TCN, model using blood glucose time series data as an input and using a predicted blood glucose value of a preset prediction horizon as an output, wherein the LSTM-TCN model comprises at least two LSTM layers and one TCN layer which are sequentially connected;
a training unit, configured to construct a training data set according to the preprocessed data, and use the training data set to train the LSTM-TCN model to obtain a trained hypoglycemia prediction model;
a prediction unit, configured to acquire blood glucose time series monitoring data, and use the hypoglycemia prediction model to predict a blood glucose value of a preset prediction horizon according to the blood glucose time series monitoring data, to obtain a predicted blood glucose value of the preset prediction horizon; and
an early warning unit, configured to perform hypoglycemia early warning according to the predicted blood glucose value of the preset prediction horizon and a preset early warning mechanism.

9. A terminal for early warning hypoglycemia, comprising a storage medium and a processor;
wherein the storage medium stores a computer execution instruction; and
the processor executes the computer execution instruction stored in the storage medium, to implement the method as claimed in any one of claims 1 to 7.

10. A computer readable storage medium, wherein the computer readable storage medium stores a computer execution instruction, the computer execution instruction, when executed by a processor, cause the processor to implement the method as claimed in any one of claims 1 to 7.
